# FASCICULE DE BREVET EUROPEEN

(11) **EP 1 415 965 B1**
(45) Date de publication et mention de la délivrance du brevet: **20.12.2006**
(21) Numéro de dépôt: 03292563.8
(22) Date de dépôt: 15.10.2003
(51) Int. Cl.: C07C 29/76

(54) **Procédé de régenération d'une solution aqueuse de glycol contenant des sels**
Verfahren zur Regenerierung einer Salze enthaltenden wässrigen Lösung von Glykol
Process for the regeneration of an aqueous solution of glycol containing salts

(30) Priorité: 28.10.2002 FR 0213425
(43) Date de publication de la demande: 06.05.2004
(73) Titulaire: Institut Français du Pétrole, 92852 Rueil-Malmaison Cedex (FR); Prosernat, 92084 Paris La Défense Cedex (FR)
(72) Inventeur: Laborie, Géraldine, 92400 Courbevoie (FR); Lecomte, Fabrice, 75020 Paris (FR); Rigaill, Chantal, 91330 Yerres (FR); Waintraub, Lionel, 94160 Saint-Mandé (FR)

(56) Documents cités:
- US-A- 4 427 507
- DATABASE WPI Section Ch, Week 199421 Derwent Publications Ltd., London, GB; Class H01, AN 1994-175166 XP002269571 & SU 1 803 173 A (GNI I PROEKTNO IZYSKATELSIJ INSTITUT "YUZHNIIGIPROGAZ") 23 mars 1993 (1993-03-23)
- DATABASE WPI Section Ch, Week 199421, Derwent Publications Ltd., London, GB; Class H01, AN 1994-175166 & SU 1 803 173 A (GNI I PROEKTNO IZYSKATELSIJ INSTITUT "YUZHNIIGIPROGAZ") 23 Mars 1993

## Description

La présente invention se rapporte au domaine technique de la régénération d'une solution aqueuse de glycol contenant des sels, et plus particulièrement du monoethylène glycol (MEG) utilisé pour le transport du gaz naturel.

Le gaz naturel en sortie des puits de production est souvent associé à de l'eau de gisement contenant des sels dissous (chlorures de sodium, chlorures de potassium, chlorures de calcium, bicarbonates de sodium, etc.). Le gaz naturel est transporté du lieu de production vers un lieu de traitement par circulation dans des conduites. Dans le cas où le gaz naturel est saturé en eau et en équilibre avec une phase aqueuse, en fonction des conditions de transport (pression et température), il peut se former des bouchons d'hydrates conduisant à l'arrêt de la production. Pour éviter ces problèmes, un inhibiteur d'hydrates tel le glycol, est injecté dans les conduites de transport. On peut utiliser une solution aqueuse contenant entre 60% et 90% poids de glycol. Après le transport, un mélange composé d'eau de gisement et de glycol est récupéré, puis traité dans une unité de régénération du glycol afin de re-concentrer le glycol, c'est à dire éliminer l'eau. Le glycol régénéré peut à nouveau être injecté dans les conduites de transport du gaz naturel.

SU 1 803 173 décrit un procédé pour enlever des sels solubles des solutions de glycol par l'addition d'un agent de déssalement.

Des systèmes de distillation du glycol pour séparer le glycol du mélange eau et glycol sont connus de l'homme du métier. En général, les systèmes de l'art antérieur permettent d'obtenir une solution aqueuse contenant entre 70% et 90% de glycol.

Cependant, la régénération du glycol conduit à concentrer les sels, présents au départ dans l'eau de gisement, dans le glycol régénéré. La concentration de sel est à l'origine de problèmes opératoires tels que l'accumulation de sels sur certaines parties du dispositif de régénération réduisant ainsi son efficacité et tels que des problèmes de corrosion du dispositif de régénération.

La présente invention propose un procédé de régénération d'une solution aqueuse de glycol permettant d'éliminer une partie de l'eau, ainsi que les sels.

La solubilité des sels dans les solutions aqueuses de glycol varie avec la température et la teneur en eau de la solution. En ajustant la température et en abaissant la teneur en eau, on abaisse la solubilité des sels dans les solutions aqueuses de glycol. Ainsi, on peut provoquer la précipitation des sels, puis séparer les sels précipités de la solution aqueuse de glycol.

De manière générale, l'invention concerne un procédé de régénération d'une solution de glycol contenant de l'eau, des hydrocarbures et des sels dissous, comportant les étapes:
a) on détend ladite solution pour libérer des hydrocarbures et pour obtenir une solution pauvre en hydrocarbures,
b) on distille dans une colonne de distillation la solution pauvre en hydrocarbures obtenue à l'étape a) pour obtenir une solution enrichie en glycol et une vapeur contenant de l'eau et des hydrocarbures;
c) on met sous vide une première partie de la solution enrichie en glycol obtenue à l'étape b) sous une pression inférieure à 90 000 Pa absolu pour obtenir de l'eau vaporisée et une solution de glycol contenant des sels précipités,
d) on sépare les sels précipités de la solution de glycol obtenue à l'étape c) pour obtenir des sels précipités et une solution de glycol appauvrie en sels.

Le procédé selon l'invention peut également comporter les étapes suivantes:
e) on met sous vide la solution de glycol appauvrie en sels obtenue à l'étape d) sous une pression inférieure à 50 000 Pa absolu pour obtenir de l'eau vaporisée et une solution de glycol contenant des sels précipités,
f) on sépare les sels précipités de la solution de glycol obtenue à l'étape e) pour obtenir des sels précipités et une deuxième solution de glycol appauvrie en sels.

A l'étape d), on peut séparer les sels précipités de la solution de glycol au moyen d'au moins une des techniques: filtration, centrifugation, séparation par ultrason.

Avant l'étape c), on peut refroidir ou chauffer la solution enrichie en glycol obtenue à l'étape b) à une température comprise entre 30°C et 150°C.

A l'étape a), on peut détendre ladite solution à une pression comprise entre 0,1 MPa et 2 MPa absolu, et à l'étape b), on peut distiller à pression atmosphérique.

La solution de glycol appauvrie obtenue à l'étape e) peut réchauffer la solution pauvre en hydrocarbures obtenue à l'étape a).

Le procédé selon l'invention peut comporter les étapes:
g) on refroidit la vapeur contenant de l'eau et des hydrocarbures obtenue à l'étape b) pour obtenir de la vapeur d'eau, une phase hydrocarbures liquides et une phase aqueuse,
h) une partie de la phase aqueuse obtenue à l'étape g) est envoyée en tête de la colonne de distillation,
i) on introduit l'eau vaporisée obtenue à l'étape c) dans ladite colonne de distillation,
j) on réunit une deuxième partie de la solution enrichie en glycol obtenue à l'étape b) et la solution de glycol appauvrie en sels obtenue à l'étape d),
k) on introduit de l'eau dans la solution de glycol appauvrie en sels obtenue à l'étape d).

Le glycol peut consister en un composé choisi dans le groupe comportant le monoéthylène glycol, le diéthylène glycol, le triéthylène glycol et le tétraéthylène glycol. Les sels peuvent comprendre au moins un des composés suivants: chlorure de sodium, chlorure de potassium, chlorure de calcium et bicarbonate de sodium, sulfate de sodium, sulfate de potassium, sulfate de calcium.

D'autres caractéristiques et avantages de l'invention seront mieux compris et apparaîtront clairement à la lecture de la description faites ci-après à titre d'exemple en se référant aux dessins parmi lesquels :
- la figure 1 représente schématiquement le procédé selon l'invention,
- les figures 2 et 3 représentent schématiquement des variantes du procédé selon l'invention.

Un gaz naturel issu d'un puits de production pétrolière est transporté par circulation dans des conduites jusqu'à une unité de traitement, par exemple une unité de déshydratation, de désacidification et/ou de dégazolinage. Pour éviter la formation d'hydrate, on injecte du glycol dans les conduites transportant le gaz naturel. Avant d'être traité, le gaz naturel sous forme gazeuse est séparé de la solution aqueuse de glycol, par exemple au moyen d'un ballon de séparation.

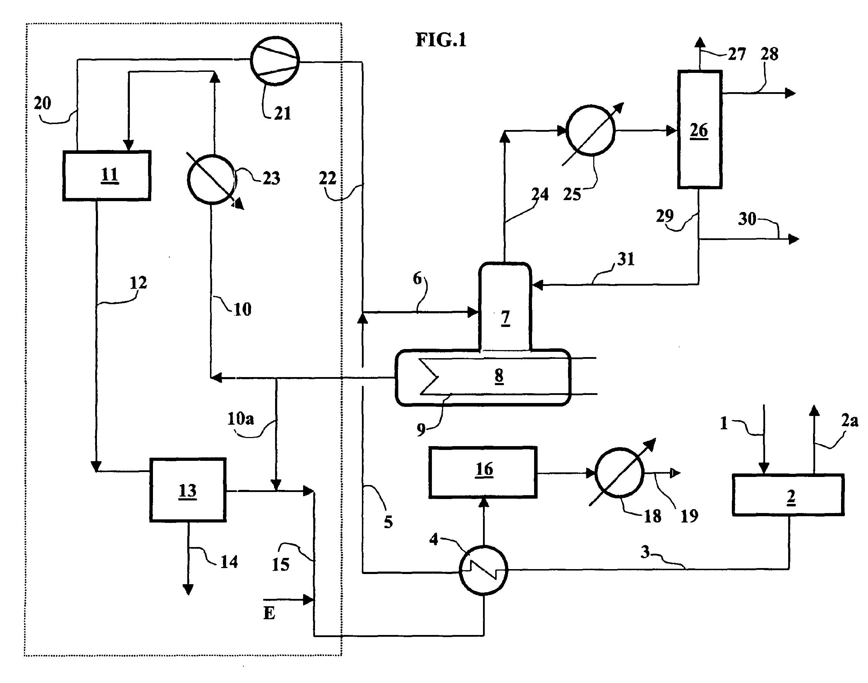

Sur la figure 1, la solution aqueuse de glycol arrive par le conduit (1). Le glycol (1) chargé en eau et en sels est introduit dans un ballon de flash (2) où il libère par le conduit (2a) les hydrocarbures coabsorbés lors du contact avec le gaz naturel. Le ballon (2) peut fonctionner entre 0,1 MPa et 2 MPa absolu, de préférence entre 0,1 et 0,6 MPa absolu.

Le glycol détendu évacué par le conduit (3) est réchauffé dans un échangeur (4) avant d'être introduit par l'intermédiaire des conduits (5) et (6) dans une colonne de régénération (7). La colonne (7) consiste en une colonne de distillation pourvue d'un rebouilleur (8) en fond de colonne et d'un conduit (31) amenant un liquide de reflux en tête de colonne. La colonne (7) est munie de plateaux, de garnissages en vrac ou de garnissages structurés. La colonne (7) peut fonctionner à pression atmosphérique. Grâce à la chaleur de rebouillage apportée par l'élément chauffant (9) et à l'effet de distillation opéré dans la colonne (7), une vapeur est produite en tête de colonne, principalement constituée d'eau et dans une proportion plus faible d'hydrocarbures.

La vapeur est évacuée de la colonne (7) par le conduit (24). Après refroidissement de cette vapeur dans un échangeur de chaleur (25), on sépare dans le ballon (26) de la vapeur évacuée par le conduit (27), une phase hydrocarbure liquide évacuée par le conduit (28) et une phase aqueuse évacuée par le conduit (29). Une partie de la phase aqueuse est envoyée par le conduit (31) dans la colonne (7) pour reflux, la partie restante étant évacuée par le conduit (30).

Le glycol récupéré en fond du rebouilleur (8) est chauffé ou refroidi dans l'échangeur (23) avant d'être introduit dans la capacité (11) par l'intermédiaire du conduit (10). L'échangeur (23) permet de régler la température, par exemple entre 30°C et 150°C, à laquelle les sels sont susceptibles de précipiter. Une partie du glycol circulant dans le conduit (10) peut être soutirée par le conduit (10a), puis mélangée avec la solution circulant dans le conduit (15). Le glycol s'écoule du rebouilleur (8) vers la capacité (11) par différence de pression. La capacité (11) est reliée à la pompe à vide (21) par le conduit (20). La pompe à vide (21) permet de réaliser une mise sous vide de la capacité (11), c'est à dire à maintenir la capacité (11) à une pression inférieure à la pression atmosphérique, par exemple inférieure à 90 000 Pa absolu, de préférence inférieure à 50 000 Pa absolu ou 20 000 Pa absolu. La pression de mise sous vide est choisie en fonction, notamment, des quantités de sels dissous et d'eau contenus dans la solution de glycol, de la température de la solution de glycol et de la nature des sels. Dans la capacité (11), sous l'effet de l'ajustement de température et de la mise sous vide, une partie de l'eau se vaporise avec un peu de glycol. L'eau vaporisée dans la capacité (11) est pompée par la pompe (21), puis est envoyée par le conduit (22) dans le rebouilleur (8) de la colonne de régénération. L'abaissement de la teneur en eau, couplé à l'ajustement de la température, permet la précipitation des sels dissous dans la solution aqueuse de glycol au niveau de la capacité (11).

La solution contenant les sels cristallisés est évacuée de la capacité (11) par le conduit (12) et est envoyée vers un dispositif de séparation (13). Le mélange peut s'écouler par gravité de la capacité (11) dans le dispositif de séparation (13) grâce à la différence de niveau entre ces deux éléments. Plus la pression dans la capacité (11) est basse, plus la différence de niveau entre la capacité (11) et le dispositif de séparation (13) est grande. Le dispositif (13) sépare les sels précipités du reste de la solution. Les sels sont récupérés par le conduit (14). La solution aqueuse de glycol débarrassée d'une partie de ses sels est évacuée du dispositif (13) par le conduit (15).

Les sels restants dans la solution circulant dans le conduit (15) sont en équilibres, c'est à dire qu'une modification des conditions thermodynamiques pourrait provoquer une précipitation de ces sels. Pour éviter la précipitation des sels dans le conduit (15), on peut y injecter de l'eau par l'intermédiaire du conduit (E). On peut également introduire une partie du glycol circulant dans le conduit (10) dans le conduit (15) par l'intermédiaire du conduit (10 bis). Ainsi, les sels restants ne sont pas susceptibles de précipiter car la teneur en eau de la solution circulant dans le conduit (15) a été augmentée.

La solution circulant dans le conduit (15) est chauffée ou refroidie dans l'échangeur de chaleur (4) avant d'être stockée dans la capacité (16). La solution de glycol est évacuée de la capacité (16) par le conduit (19) après un éventuel refroidissement dans l'échangeur (18), puis est injectée dans un conduit transportant un gaz naturel issu d'un puits de production.

Le dispositif de séparation (13) peut consister en un dispositif de filtration (filtration sur media filtrant ou sur précouche), un dispositif de centrifugation (utilisation de centrifugeuse ou de cyclone), un dispositif de séparation par ultrason ou une association des ces techniques.

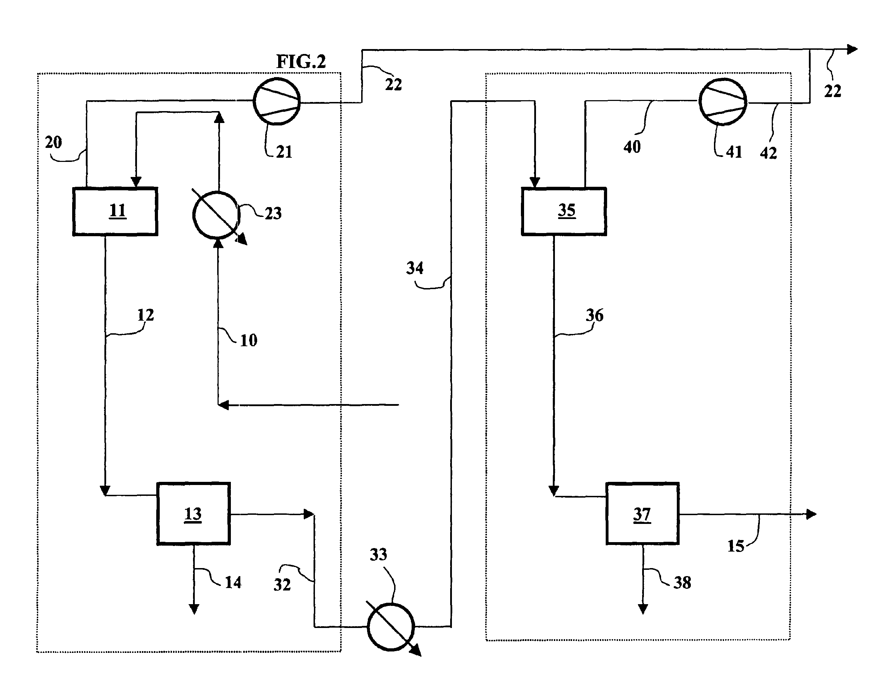

La figure 2 présente une variante d'une partie du procédé selon la figure 1. Le procédé selon la figure 2 remplace la partie du procédé de la figure 1 contenue dans le rectangle en pointillés. Sur les figures 1 et 2, les références identiques désignent les mêmes éléments.

La partie de procédé représentée par la figure 2 comportent deux étapes de précipitation et de séparation des sels mises en série.

Le glycol récupéré en fond de la colonne de régénération arrive par le conduit (10), par exemple sous pression atmosphérique. Il est réchauffé ou refroidi, par exemple à une température comprise entre 30°C et 150°C, dans l'échangeur de chaleur (23) puis est introduit dans la capacité (11). La capacité (11) est reliée à la pompe à vide (21) par le conduit (20). La pompe à vide (21) permet de réaliser une mise sous vide de la capacité (11) en maintenant la capacité (11) à une pression inférieure à 90 000 Pa absolu, de préférence inférieure à 50 000 Pa. L'eau vaporisée dans la capacité (11) est pompée par la pompe (21), puis est envoyée par le conduit (22) dans le rebouilleur (8) de la colonne de régénération (7). La solution contenant les sels cristallisés est évacuée de la capacité (11) par le conduit (12), et est envoyée vers un dispositif de séparation (13). Le dispositif (13) sépare les sels précipités du reste de la solution. Les sels sont récupérés par le conduit (14). La solution aqueuse de glycol au moins en partie débarrassée de ses sels est évacuée du dispositif (13) par le conduit (32).

La solution circulant dans le conduit (32) est réchauffée ou refroidie, par exemple à une température comprise entre 30°C et 150°C, par l'échangeur de chaleur (33), puis est envoyée par le conduit (34) dans la capacité (35). La capacité (35) est reliée à la pompe à vide (41) par le conduit (40). La pompe à vide (41) permet de réaliser une mise sous vide de la capacité (35), en maintenant la capacité (35) à une pression inférieure à 50 000 Pa absolu, de préférence inférieure à 20 000 Pa. L'eau vaporisée dans la capacité (35) est pompée par la pompe (41), puis est envoyée par les conduits (42) et (22) dans le rebouilleur (8) de la colonne de régénération. Le mélange de sels cristallisés et de la solution est évacué de la capacité (35) par le conduit (36), et est envoyé vers un dispositif de séparation (37). Le dispositif (37) sépare les sels précipités du reste de la solution. Les sels sont récupérés par le conduit (38). La solution aqueuse de glycol en partie débarrassée de ses sels est évacuée du dispositif (37) par le conduit (15) vers la capacité de stockage (16). Ce dispositif particulier permet de réduire de façon plus poussée la quantité résiduelle de sels présents dans la solution de glycol régénéré.

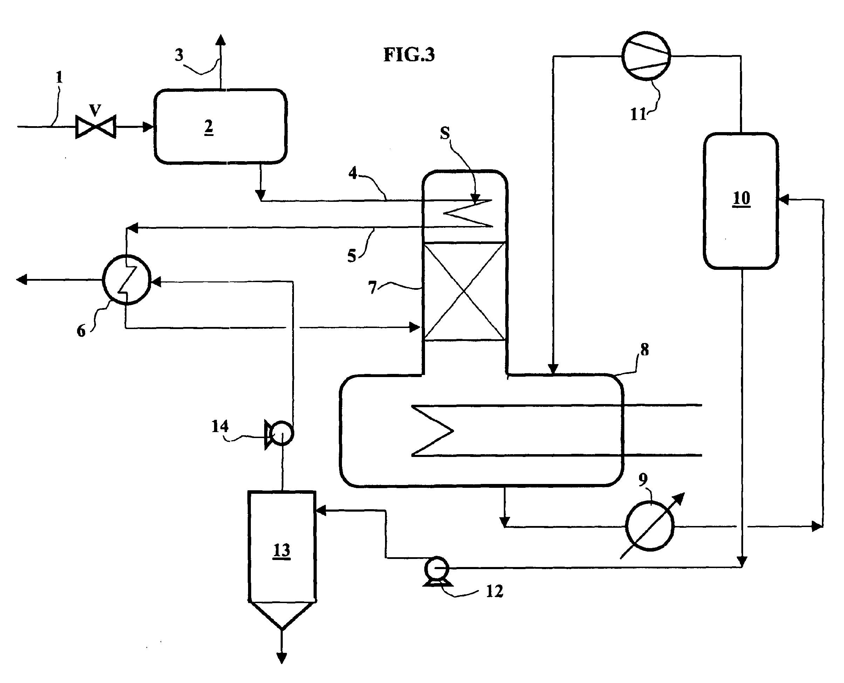

Un exemple numérique des conditions de fonctionnement du procédé selon l'invention est donné en relation avec le procédé schématisé par la figure 3.

Un glycol riche (MEG + eau + sels) arrivant par le conduit (1) doit être traité afin de le concentrer à 90 % poids et afin d'éliminer une partie des sels qu'il contient de manière à éviter des dépôts de sels sur certains éléments de la régénération et à limiter les phénomènes de corrosion. Le débit de glycol riche à traiter est de 12 500 kg/h, sa concentration est de 55 % poids de MEG et il contient 0,3 % poids de sels. Il est disponible à 30 °C et à 0,6 MPa absolu.

Le glycol riche (1) est détendu à 0,5 MPa absolu par la vanne (V), puis est envoyé dans un ballon de flash (2) où les hydrocarbures dissous dans le glycol sont vaporisés, puis évacués par le conduit (3). Le glycol riche issu du ballon (2) par le conduit (4) est réchauffé dans le serpentin (S) de reflux de la colonne de régénération (7), puis est introduit par le conduit (5) dans l'échangeur (6) pour être chauffé jusqu'à une température d'environ 75°C, puis alimente la colonne de régénération (7). Le rebouilleur (8) fonctionne à pression atmosphérique à une température de 116,5°C, le glycol est alors concentré à 70 % poids et dans ces conditions, il n'y a pas ou peu de risque de précipitation des sels dans le rebouilleur. Le glycol partiellement régénéré est ensuite chauffé à une température de 131°C dans l'échangeur (9) et introduit dans la capacité (10). La capacité (10) est maintenue sous vide à une pression de 80 000 Pa absolu. Les conditions de fonctionnement de cette capacité ont été choisies de manière à concentrer le glycol jusqu'à 90 % poids et à faire précipiter les sels. L'eau vaporisée dans la capacité (10) est pompée par la pompe (11) et renvoyée dans le rebouilleur (8). La solution de glycol concentrée à 90 % poids et contenant des sels cristallisés est envoyée par la pompe (12) vers un dispositif de séparation par centrifugation (13). Le glycol régénéré et débarrassé de ses sels est ensuite pompé (14) et envoyé dans l'échangeur (6) afin d'être refroidi par échange thermique avec le glycol circulant dans le conduit (5).

## Revendications

1. Procédé de régénération d'une solution de glycol contenant de l'eau, des hydrocarbures et des sels dissous, comportant les étapes:
a) on détend ladite solution pour libérer des hydrocarbures et pour obtenir une solution pauvre en hydrocarbures,
b) on distille dans une colonne de distillation la solution pauvre en hydrocarbures obtenue à l'étape a) pour obtenir une solution enrichie en glycol et une vapeur contenant de l'eau et des hydrocarbures,
c) on met sous vide une première partie de la solution enrichie en glycol obtenue à l'étape b) sous une pression inférieure à 90 000 Pa absolu pour obtenir de l'eau vaporisée et une solution de glycol contenant des sels précipités,
d) on sépare les sels précipités de la solution de glycol obtenue à l'étape c) pour obtenir des sels précipités et une solution de glycol appauvrie en sels.

2. Procédé selon la revendication 1, comportant les étapes:
e) on met sous vide la solution de glycol appauvrie en sels obtenue à l'étape d) sous une pression inférieure à 50 000 Pa absolu pour obtenir de l'eau vaporisée et une solution de glycol contenant des sels précipités,
f) on sépare les sels précipités de la solution de glycol obtenue à l'étape e) pour obtenir des sels précipités et une deuxième solution de glycol appauvrie en sels.

3. Procédé selon l'une des revendications précédentes dans lequel, à l'étape d), on sépare les sels précipités de la solution de glycol au moyen d'au moins une des techniques: filtration, centrifugation, séparation par ultrason.

4. Procédé selon l'une des revendications précédentes, dans lequel avant l'étape c), on refroidit la solution enrichie en glycol obtenue à l'étape b) à une température comprise entre 30°C et 150°C.

5. Procédé selon l'une des revendications 1 à 3, dans lequel avant l'étape c), on chauffe la solution enrichie en glycol obtenue à l'étape b) à une température comprise entre 30°C et 150°C.

6. Procédé selon l'une des revendications précédentes, dans lequel à l'étape a), on détend ladite solution à une pression comprise entre 0,1 MPa et 2 MPa absolu, et dans lequel à l'étape b), on distille à pression atmosphérique.

7. Procédé selon l'une des revendications précédentes, dans lequel la solution de glycol appauvrie obtenue à l'étape e) réchauffe la solution pauvre en hydrocarbures obtenue à l'étape a).

8. Procédé selon l'une des revendications précédentes, dans lequel on effectue les étapes:
g) on refroidit la vapeur contenant de l'eau et des hydrocarbures obtenue à l'étape b) pour obtenir de la vapeur d'eau, une phase hydrocarbures liquides et une phase aqueuse,
h) une partie de la phase aqueuse obtenue à l'étape g) est envoyée en tête de la colonne de distillation.

9. Procédé selon l'une des revendications précédentes, dans lequel on effectue l'étape:
i) on introduit l'eau vaporisée obtenue à l'étape c) dans ladite colonne de distillation.

10. Procédé selon l'une des revendications précédentes, dans lequel on effectue l'étape:
j) on réunit une deuxième partie de la solution enrichie en glycol obtenue à l'étape b) et la solution de glycol appauvrie en sels obtenue à l'étape d).

11. Procédé selon l'une des revendications 1 à 10, dans lequel on effectue l'étape:
k) on introduit de l'eau dans la solution de glycol appauvrie en sels obtenue à l'étape d).

12. Procédé selon l'une des revendications précédentes dans lequel le glycol consiste en un composé choisi dans le groupe comportant le monoéthylène glycol, le diéthylène glycol, le triéthylène glycol et le tétraéthylène glycol.

13. Procédé selon l'une des revendications précédentes dans lequel les sels comprennent au moins un des composés suivants: chlorure de sodium, chlorure de potassium, chlorure de calcium et bicarbonate de sodium, sulfate de sodium, sulfate de potassium, sulfate de calcium.

## Claims

1. A method for regenerating a glycol solution containing water, hydrocarbons and dissolved salts, comprising the following steps:
a) expanding said solution to free hydrocarbons and to obtain a solution which is low in hydrocarbons,
b) distilling, in a distillation column, the solution which is low in hydrocarbons obtained in step a) to obtain a glycol-enriched solution and a vapour containing water and hydrocarbons,
c) placing a first part of the glycol-enriched solution obtained in step b) under vacuum at a pressure lower than 90000 absolute Pa to obtain vaporised water and a glycol solution containing precipitated salts,
d) separating the precipitated salts from the glycol solution obtained in step c) to obtain precipitated salts and a glycol solution depleted in salts.

2. The method according to Claim 1, comprising the following steps:
e) placing the glycol solution depleted in salts obtained in step d) under vacuum at a pressure lower than 50000 absolute Pa to obtain vaporised water and a glycol solution containing precipitated salts,
f) separating the precipitated salts from the glycol solution obtained in step e) to obtain precipitated salts and a second glycol solution depleted in salts.

3. The method according to one of the preceding claims, in which, in step d), the precipitated salts are separated from the glycol solution by means of at least one of the following techniques: filtration, centrifuging, separation by ultrasound.

4. The method according to one of the preceding claims, in which, prior to step c), the glycol-enriched solution obtained in step b) is cooled to a temperature of between 30°C and 150°C.

5. The method according to one of Claims 1 to 3, in which, prior to step c), the glycol-enriched solution obtained in step b) is heated to a temperature of between 30°C and 150°C.

6. The method according to one of the preceding claims, in which, in step a), said solution is expanded to a pressure of between 0.1 absolute MPa and 2 absolute MPa, and in which, in step b), distillation is carried out at atmospheric pressure.

7. The method according to one of the preceding claims, in which the depleted glycol solution obtained in step e) heats the solution which is low in hydrocarbons obtained in step a).

8. The method according to one of the preceding claims, in which the following steps are carried out:
g) cooling the vapour containing water and hydrocarbons obtained in step b) to obtain water vapour, a liquid hydrocarbon phase and an aqueous phase,
h) a part of the aqueous phase obtained in step g) is sent to the top of the distillation column.

9. The method according to one of the preceding claims in which the following step is carried out:
i) introducing the vaporised water obtained in step c) into said distillation column.

10. The method according to one of the preceding claims in which the following step is carried out:
j) combining a second part of the glycol-enriched solution obtained in step b) and the glycol solution depleted in salts obtained in step d).

11. The method according to one of Claims 1 to 10, in which the following step is carried out:
k) introducing water into the glycol solution depleted in salts obtained in step d).

12. The method according to one of the preceding claims in which the glycol consists of a compound chosen from the group comprising monoethylene glycol, diethylene glycol, triethylene glycol and tetraethylene glycol.

13. The method according to one of the preceding claims in which the salts include at least one of the following compounds: sodium chloride, potassium chloride, calcium chloride and sodium bicarbonate, sodium sulphate, potassium sulphate, calcium sulphate.

## Patentansprüche

1. Verfahren zur Regenerierung einer Wasser, Kohlenwasserstoffe und gelöste Salze enthaltenden Glykollösung, das die folgenden Schritte umfasst:
a) Expandieren der Lösung, um Kohlenwasserstoffe freizusetzen und um eine kohlenwasserstoffarme Lösung zu erhalten,
b) Destillieren der im Schritt a) erhaltenen kohlenwasserstoffarmen Lösung in einer Destillationskolonne, um eine glykolangereicherte Lösung und einen Wasser und Kohlenwasserstoffe enthaltenden Dampf zu erhalten,
c) Vakuumbehandeln eines ersten Teils der im Schritt b) erhaltenen glykolangereicherten Lösung bei einem Druck, der niedriger als 90 000 Pa absolut ist, um Wasserdampf und eine ausgefällte Salze enthaltende Glykollösung zu erhalten,
d) Abtrennen der aus der im Schritt c) erhaltenen Glykollösung ausgefällten Salze, um ausgefällte Salze und eine Glykollösung mit vermindertem Salzgehalt zu erhalten.

2. Verfahren nach Anspruch 1, das außerdem die folgenden Schritte umfasst:
e) Vakuumbehandeln der im Schritt d) erhaltenen Glykollösung mit vermindertem Salzgehalt bei einem Druck, der niedriger als 50 000 Pa absolut ist, um Wasserdampf und eine ausgefällte Salze enthaltende Glykollösung zu erhalten,
f) Abtrennen der aus der im Schritt e) erhaltenen Glykollösung ausgefällten Salze, um ausgefällte Salze und eine zweite Glykollösung mit vermindertem Salzgehalt zu erhalten.

3. Verfahren nach einem der vorhergehenden Ansprüche, bei dem im Schritt d) die aus der Glykollösung ausgefällten Salze mit Hilfe mindestens einer der Techniken der Filtration, der Zentrifugation und der Ultraschallseparation abgetrennt werden.

4. Verfahren nach einem der vorhergehenden Ansprüche, bei dem vor dem Schritt c) die im Schritt b) erhaltene glykolangereicherte Lösung auf eine Temperatur im Bereich zwischen 30 °C und 150 °C abgekühlt wird.

5. Verfahren nach einem der Ansprüche 1 bis 3, bei dem vor dem Schritt c) die im Schritt b) erhaltene glykolangereicherte Lösung auf eine Temperatur im Bereich zwischen 30 °C und 150 °C erwärmt wird.

6. Verfahren nach einem der vorhergehenden Ansprüche, bei dem im Schritt a) die Lösung bei einem Druck im Bereich zwischen 0,1 MPa und 2 MPa absolut expandiert wird und bei dem im Schritt b) bei atmosphärischem Druck destilliert wird.

7. Verfahren nach einem der vorhergehenden Ansprüche, bei dem die im Schritt e) erhaltene Lösung mit vermindertem Glykolgehalt die im Schritt a) erhaltene kohlenwasserstoffarme Lösung erwärmt.

8. Verfahren nach einem der vorhergehenden Ansprüche, bei dem außerdem die folgenden Schritte ausgeführt werden:
g) Abkühlen des im Schritt b) erhaltenen Dampfes, der Wasser und Kohlenwasserstoffe enthält, um Wasserdampf, eine Phase flüssiger Kohlenwasserstoffe und eine wässrige Phase zu erhalten,
h) Einleiten eines Teils der im Schritt g) erhaltenen wässrigen Phase in den Kopf der Destillationskolonne.

9. Verfahren nach einem der vorhergehenden Ansprüche, bei dem außerdem der folgende Schritt ausgeführt wird:
i) Einleiten des im Schritt c) erhaltenen Wasserdampfes in die Destillationskolonne.

10. Verfahren nach einem der vorhergehenden Ansprüche, bei dem außerdem der folgende Schritt ausgeführt wird:
j) Vereinigen des zweiten Teils der im Schritt b) erhaltenen glykolangereicherten Lösung und der im Schritt d) erhaltenen Glykollösung mit vermindertem Salzgehalt.

11. Verfahren nach einem der vorhergehenden Ansprüche 1 bis 10, bei dem außerdem der folgende Schritt ausgeführt wird:
k) Einleiten von Wasser in die im Schritt d) erhaltene Glykollösung mit vermindertem Salzgehalt.

12. Verfahren nach einem der vorhergehenden Ansprüche, bei dem das Glykol aus einer Verbindung besteht, die aus der Gruppe gewählt ist, die Monoethylenglykol, Diethylenglykol, Triethylenglykol und Tetraethylenglykol umfasst.

13. Verfahren nach einem der vorhergehenden Ansprüche, bei dem die Salze mindestens eine der folgenden Verbindungen enthalten: Natriumchlorid, Kaliumchlorid, Calciumchlorid und Natriumhydrogencarbonat, Natriumsulfat, Kaliumsulfat, Calciumsulfat.
